Europäisches Patentamt

European Patent Office

Office européen des brevets

(11) Veröffentlichungsnummer: **0 033 057**
**A1**

## (12) EUROPÄISCHE PATENTANMELDUNG

(21) Anmeldenummer: **80810041.6**

(22) Anmeldetag: **05.02.80**

(51) Int. Cl.³: **A 63 B 71/10**
**A 42 B 3/00, A 61 F 9/04**

(43) Veröffentlichungstag der Anmeldung:
**05.08.81 Patentblatt 81/31**

(84) Benannte Vertragsstaaten:
**AT BE DE FR GB IT NL SE**

(71) Anmelder: **Hediger, Peter**
**Büelhofstrasse 358**
**CH-8185 Rüti-Bülach(CH)**

(72) Erfinder: **Hediger, Peter**
**Büelhofstrasse 358**
**CH-8185 Rüti-Bülach(CH)**

(74) Vertreter: **Feldmann, Clarence Paul**
**c/o Patentanwaltsbüro FELDMANN AG Postfach**
**Kanalstrasse 17**
**CH-8152 Glattbrugg(CH)**

(54) **Gesichtsschutz für Sportler, insbesondere für Eishockey-Spieler.**

(57) Der Gesichtsschutz (10) verbindet die Vorteile einer völlig geschlossenen Gesichtsmaske aus durchsichtigem Kunststoff mit den Vorteilen eines Stahlgittergesichtsschutzes.

Die durchsichtige Kunststoffscheibe (11) ist mit mehreren Reihen von Durchbrüchen (12) versehen. Der Abstand der Stege (13) ist kleiner als die Höhe der Schaufel eines Eishockeystockes. Mittels je zwei Schrauben (4,5) ist der Schutz (10) am Helm (11) lösbar befestigt.

FIG.1

EP 0 033 057 A1

Croydon Printing Company Ltd.

Peter Hediger
8185 Rüti-Bülach

Gesichtsschutz für Sportler, insbesondere

für Eishockey-Spieler

Die vorliegende Erfindung betrifft einen Gesichtsschutz
aus durchsichtigem Material, der fest oder lösbar mit
einem Helm verbunden ist.

Sporthelme mit Gesichtsschutz sind schon seit längerer
Zeit, insbesondere im Eishockeysport, bekannt. Bei den
älteren Modellen ist der Gesichtsschutz aus einem Stahldrahtgeflecht gefertigt. Aus Sicherheitsgründen musste
das Drehtgeflecht relativ dicht sein, wodurch eine
Beeinträchtigung  der Sicht des Spielers nicht vermeidbar ist. Deshalb kamen in den letzten Jahren immer

hed 1/EU

mehr Helme mit einem Gesichtsschutz aus organischem Glas
auf den Markt. Diese Gesichtsschutze bestehen aus einer
völlig geschlossenen Scheibe aus Kunststoff, welche das
Gesicht des Sportlers vollständig abdeckt.

Es bestehen drei wesentliche Gründe, dass sich solche Gesichtsschutze, insbesondere im Eishockeysport, nicht
durchgesetzt haben. Zum ersten neigen vollkommen geschlossene Gesichtsschutze sehr stark zum Anlaufen, was
die Sicht des Spielers erheblich einschränkt. Zum zweiten
sind diese Gesichtsschutze beim Regen oder Schneeefall
völlig untauglich, da die auf dem Gesichtsschutz sich
niedersetzenden Tropfen ein völlig diffuses Bild verursachen. Zum dritten ist die Atemluftzufuhr für den
Sportler, bei tiefgezogenen Scheiben, völlig unzureichend.
Gegen das Erstere kann man sich durch Auftragen eines Antibeschlagmittels schützen, sofern die Scheibe nur gerade
die Augenpartie abdeckt. Bei Schutzen, die aber auch Nasen-
und Mundpartie abdecken, sind auch die besten Antibeschlagmittel nutzlos. Viele Antibeschlagmittel greifen zudem den
Kunststoff des Gesichtsschutzes an und bewirken eine
Materialveränderung, die zur Trübung und zur Sprödigkeit
des Materials führen. Bei Regen oder Schneefall müsste
ein solcher Gesichtsschutz sogar vollständig entfernt
werden. Es ist daher Aufgabe der vorliegenden Erfindung,

- 3 -

einen Gesichtsschutz für Sporthelme zu schaffen, bei
dem die vorgenannten Nachteile vermieden werden.

Diese Aufgabe löst ein Gesichtsschutz für Sportler, der
sich dadurch auszeichnet, dass er aus einer gewölbten
Kunststoffscheibe mit mehreren Durchbrüchen gefertigt
ist.

In der Zeichnung ist ein Ausführungsbeispiel des Erfindungsgegenstandes dargestellt und anhand der nachfolgenden Beschreibung erläutert: Es zeigt:

Figur 1   den erfindungsgemässen Gesichtsschutz
          von der Seite und

Figur 2   denselben Gesichtsschutz in der Ansicht
          von vorne.

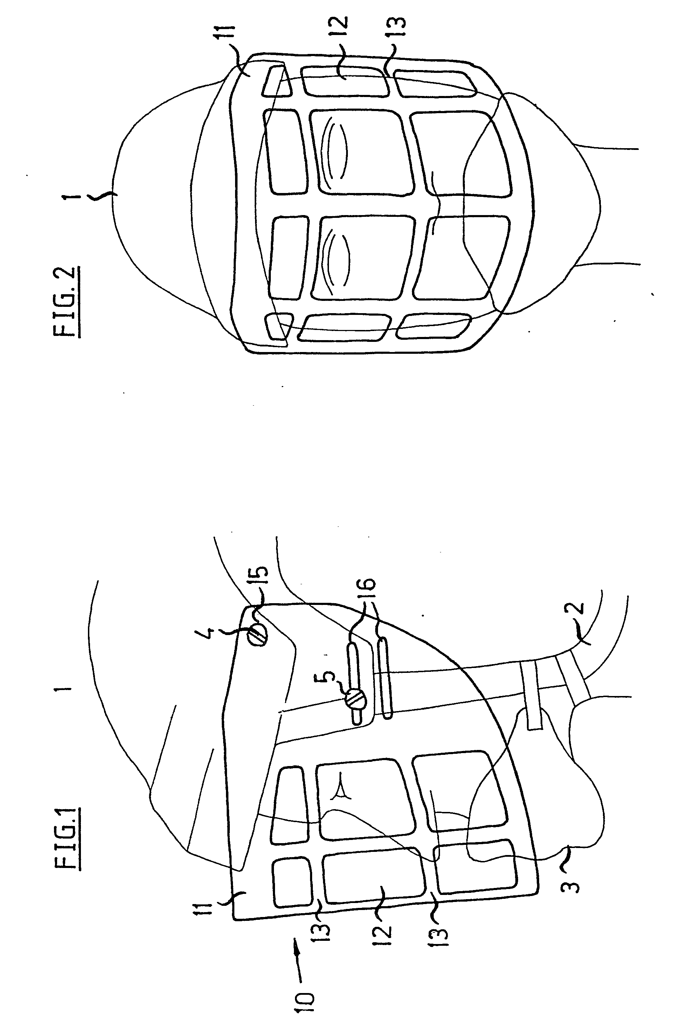

Mit 1 ist ein handelsüblicher Kunststoff-Eishockeyhelm
bezeichnet. Der Helm 1 wird durch einen Kinnriemen 2,
an dem ein Kinnschutz 3 befestigt ist, gehalten. Zwei
Schrauben 4,5 dienen der Befestigung des Gesichtsschutzes
am Helm 1. Der Gesichtsschutz als Ganzes ist mit 10
bezeichnet und besteht aus einer gewölbten Scheibe 11.
Die Scheibe 11 besteht aus einem durchsichtigen, schlaghed 1/EU

festen und splittersicheren Kunststoff. Als Material eignet
sich besonders Polycarbonat, ein unter der Handelsbezeichnung "MAKRALON" oder "LEXAN" erhältliches Produkt. Die
Scheibe 11 wird in flachem Zustand bearbeitet und erst
nachträglich in die endgültige Form gebracht. Im flachen
Zustand weist die Scheibe 11 eine annähernd halbmondförmige
Form auf. Ueber das gesamte Gesichtsfeld sind drei Reihen
mit Durchbrüchen 12 angeordnet, die eine gute Sicht
garantieren. Die zwischen den Durchbrüchen verlaufenden
Stege 13 müssen eine Minimalbreite aufweisen, die alle
Sicherheitsvorschriften berücksichtigen, als eine optimale
Breite werden ca. 5 mm angesehen. Die maximale Oeffnungsweite 14 der Durchbrüche 12 ist von zwei Massen abhängig,
erstens vom Durchmesser des Pucks und zweitens von der
Schaufelbreite eines Eishockeystockes. Die Bohrung 15
dient als Durchgangsloch für die Schraube 4. Die beiden
annähernd parallel zur Oberkante des Gesichtsschutzes
verlaufenden Längsschlitze 16 dienen zusammen mit der
Schraube 5 zur Einstellung der Gesichtsmaske. Dank den
Abmessungen und der Anordnung dieser Schlitze kann der
Schutz an verschiedenen Helmtypen montiert werden, und
so verstellt werden, dass er sich für Spieler jeden
Alters eignet. Die Form und Anordnung der Durchbrüche
12 kann mehr

hed 1/EU

- 5 -

oder weniger frei gestaltet werden, doch sollte dabei
darauf geachtet werden, dass nicht zufällig ein Steg
13 in der optischen Achse der Augen liegt.

Die Anordnung der Durchbrüche 12 in drei zur Oberkante
des Schutzes parallel laufenden Reihen ist besonders
sinnvoll. Die mittlere Reihe dient insbesondere der
freien Sicht, wobei fast zwangsläufig kein horizontaler
Steg in der optischen Achse der Augen zu liegen kommt.
Die untere Reihe dient der Abführung der Atemluft,
während die obere Reihe gewährleistet, dass keine
Warmluft hinter dem Gesichtsschutz hängen bleibt.

Die erfindungsgemäss Gesichtsmaske kann auch fest an
einem Helm montiert sein, dies kommt insofern mehr in
Frage, als üblich, da auch bei schlechten Witterungsbedingungen keine Sichtbeeinflussung des Spielers
eintritt. Neben der gezeigten Befestigungsform
kommen selbstverständlich auch andere Befestigungsarten in Frage.

hed 1/EU

P. D. Feldmann, Dipl. Ing. ETH
C. P. Feldmann, Ing. HTL
Members of AIPPI

**0033057**
**Patentanwaltsbüro Feldmann A**

- 1 -

P a t e n t a n s p r ü c h e

1. Gesichtsschutz für Sportler, insbesondere für Eishockey-
   Spieler, aus durchsichtigem Material, der fest oder
   lösbar mit einem Helm verbunden ist, dadurch gekennzeichnet, dass der Gesichtsschutz (10) aus einer
   gewölbten Kunststoffscheibe (11) mit mehreren Durchbrüchen (12) gefertigt ist.

2. Gesichtsschutz nach Anspruch 1, dadurch gekennzeichnet,
   dass die zwischen den Durchbrüchen (12) verlaufenden
   Stege (13) ausserhalb der optischen Achse des Helmträgers
   angeordnet sind.

3. Gesichtsschutz nach Anspruch 1, dadurch gekennzeichnet,
   dass die Durchbrüche (12) in drei parallel zur Oberkante
   der Scheibe (11) verlaufenden Reihen angeordnet sind.

4. Gesichtsschutz nach Anspruch 1, dadurch gekennzeichnet,
   dass die Durchbruchsweite (14) der Durchbrüche (12)
   geringer als der Durchmesser eines Eishockeypucks,
   bzw. der Höhe der Schaufel eines Eishockeystockes ist.

hed 1/EU

- 2 -

5. Gesichtsschutz nach Anspruch 1, dadurch gekennzeichnet,
dass der Schutz mittels je zwei Schrauben (4,5) am Helm (1)
lösbar befestigt ist.

6. Gesichtsschutz nach Anspruch 1, dadurch gekennzeichnet,
dass der Schutz aus einem durchsichtigen Kunststoff
gefertigt ist.

FIG.1

1

11

4

15

10

13

5

12

16

13

3

2

FIG. 2

1

11

12

13

- 1/1 -

0033057

# EUROPÄISCHER RECHERCHENBERICHT

Europäisches Patentamt

| EINSCHLÄGIGE DOKUMENTE | | | KLASSIFIKATION DER ANMELDUNG (Int.Cl.3) |
|---|---|---|---|
| **Kategorie** | **Kennzeichnung des Dokuments mit Angabe, soweit erforderlich, der maßgeblichen Teile** | **betrifft Anspruch** | |
| | US - A - 3 897 597 (D.R. KASPER) <br> * Ansprüche 1, 4; Fig. 1 * <br> --- | 1,6 | A 63 B 71/10 <br> A 42 B 3/00 <br> A 61 F 9/04 |
| | US - A - 3 132 345 (L.F. KEITH) <br> * Anspruch 1; Fig. 1, 2 * <br> --- | 1,6 | |
| | US - A - 2 790 175 (T.J. SOWLE) <br> * Spalte 3, Zeilen 19 bis 21; <br> Fig. 1, 2 * <br> --- | 1,6 | |
| | US - A - 2 616 081 (J.N. WEAVER et al.) <br> * Ansprüche 1, 2; Fig. 1, 3 * <br> --- | 1,2, 6 | **RECHERCHIERTE SACHGEBIETE (Int. Cl.3)** <br><br> A 42 B 1/00 <br> A 42 B 3/00 |
| | US - A - 2 525 389 (H.L. ZELLER) <br> * Anspruch 1; Spalte 2, Zeilen 15 <br> bis 19 * <br> --- | 1,6 | A 61 F 9/00 <br> A 63 B 71/00 |
| A | US - A - 3 608 089 (P.A. ABBATELLI) <br> * Fig. 1 * <br> --- | 1,3 | |
| A | US - A - 3 373 443 (M.T. MARIETTA) <br> * Spalte 3, Zeilen 65 bis 70; Fig. 1 * <br> --- | 1,4 | **KATEGORIE DER GENANNTEN DOKUMENTE** |
| A | US - A - 3 319 261 (F.R. DUNNING) <br> * Fig. 1 * <br> --- | 1,2,3 | X: von besonderer Bedeutung <br> A: technologischer Hintergrund <br> O: nichtschriftliche Offenbarung <br> P: Zwischenliteratur |
| A | US - A - 3 220 014 (E. STROHM et al.) <br> * Fig. 1 * <br> ---- | 1 | T: der Erfindung zugrunde liegende Theorien oder Grundsätze <br> E: kollidierende Anmeldung <br> D: in der Anmeldung angeführtes Dokument <br> L: aus andern Gründen angeführtes Dokument |

X Der vorliegende Recherchenbericht wurde für alle Patentansprüche erstellt.

&: Mitglied der gleichen Patentfamilie, übereinstimmendes Dokument

| Recherchenort | Abschlußdatum der Recherche | Prüfer |
|---|---|---|
| Berlin | 22-09-1980 | KANAL |

EPA form 1503.1 06.78